# EUROPÄISCHE PATENTANMELDUNG

(11) **EP 2 238 966 A2**
(43) Veröffentlichungstag der Anmeldung: **13.10.2010**
(21) Anmeldenummer: 10157335.0
(22) Anmeldetag: 23.03.2010
(51) Int. Cl.: A61K 8/34, A61K 8/362, A61K 8/44, A61K 8/64, A61Q 5/12, A61K 8/46

(54) **Kopfhautschonende Shampoos und Conditioner**

(30) Priorität: 08.04.2009 DE 102009002285
(71) Anmelder: Henkel AG & Co. KGaA, 40589 Düsseldorf (DE)
(72) Erfinder: Kleen, Astrid, 20457, Hamburg (DE)

(57) **Zusammenfassung**

Haarreinigungs - und -konditioniermittel, die den mit ihnen behandelten Haaren vorteilhafte Eigenschaften verleihen und dabei besonders die Steigerung des Haarvolumens und der Haarfülle sowie die Kopfhautverträglichkeit fördern, enthalten in einem kosmetisch akzeptablen Träger - bezogen auf ihr Gewicht -
a) mindestens ein oberflächenaktives Mittel, ausgewählt aus der Gruppe der anionischen, amphoteren, zwitterionischen, nichtionischen und/oder kationischen Tenside,
b) mindestens einen geradkettigen oder zyklischen, verzweigten oder unverzweigten aliphatischen C₂-C₆-Alkohol, der an 2 bis 6 Kohlenstoffatomen jeweils eine Hydroxygruppe trägt, und
c) mindestens ein funktionalisiertes Tetrapeptid.

## Beschreibung

Die vorliegende Erfindung betrifft Haarbehandlungsmittel, basierend auf einer speziellen Kombination aus einem funktionalisierten Tetrapeptid, einem Tensid und einem Alkohol sowie die Verwendung des Mittels zur Steigerung der Haarkonditionierenden Eigenschaften und zur Erhaltung bzw. Verstärkung eines neutralen Blondtons.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten.

Nicht zuletzt durch die starke Beanspruchung der Haare, beispielsweise durch das Färben oder Dauerwellen, durch die häufige Reinigung der Haare mit Shampoos und anschließendes Föhnen mit Föhnhitze oder durch Umweltbelastungen, nimmt die Bedeutung von Pflegeprodukten mit möglichst lang anhaltender Wirkung zu. Der Grund dafür ist, dass die Haare durch die Behandlung/Beanspruchung sowohl äußerlich, als auch in Ihrer Struktur geschädigt werden können, was ihnen ein unattraktives Ansehen verleiht, das sich durch mangelnde Glätte, Weichheit, mangelnden Glanz, aber auch durch eine schlechtere Kämmbarkeit, Haarbruch oder Spliss bemerkbar machen kann. Neben den Haaren kann auch die Kopfhaut unter den vorgenannten Bedingungen leiden und trocken und rissig werden mit der Folge, dass Schuppenbildung und Juckreiz auftreten kann.

Daher ist es seit langem üblich, die Haare einer speziellen Nachbehandlung zu unterziehen, um den Haaren und der Kopfhaut Pflegestoffe zuzuführen, die den Haaren wieder ein schönes äußeres Aussehen verleihen und die Haarstruktur kräftigen sowie die Kopfhaut pflegen bzw. vor dem Austrocknen schützen. Bei solchen Nachbehandlungen werden, üblicherweise in Form einer Spülung, die Haare mit speziellen Wirkstoffen, beispielsweise quaternären Ammoniumsalzen oder speziellen Polymeren, behandelt. Durch diese Behandlung können, je nach Formulierung, die Kämmbarkeit, der Halt, die Fülle sowie der Glanz der Haare verbessert, und die Splissrate verringert werden.

Trotz der mannigfaltigen Bemühungen der Haarpflege- und -regeneration in den letzten Jahrzehnten stellen sich immer neue Problemstellungen bei der Haarbehandlung, für die die Fachwelt fortwährend an neuen Lösungen arbeiten muss.

So wurde festgestellt, dass die Haare durch Färben, Dauerwellen oder Blondieren und anschließendes Waschen und Konditionieren fühlbar an Volumen und Haarfülle verlieren können, so dass die Reinigung- und Konditionierung (die nach einem Färbe-, Bleich- oder Dauerwellvorgang unbedingt erforderlich ist) auch erhebliche Nachteile für die Ästhetik der Haare mit sich bringt. Des Weiteren wird die Kopfhaut durch die vorgenannten Prozesse ebenfalls mit beansprucht und sensible Kopfhaut leidet unter den häufigen Behandlungen und es kann der Fall eintreten, dass die Haut noch sensibler wird oder die Sensibilität von ohnehin bereits sensibler Kopfhaut weiter verstärkt wird, so dass das Irritationspotential der Kopfhaut immer weiter erhöht wird. Dies kann im Extremfall zur Folge haben, dass das Allergiepotential der Kopfhaut zunimmt und sie nicht mehr dazu in der Lage ist, Haar- und Kopfhautpflegende Wirkstoffe aufzunehmen.

Aufgabe der vorliegenden Erfindung ist daher die Bereitstellung reinigender und/oder konditionierender Haarbehandlungsmittel, die das Haarvolumen und die Haarfülle insbesondere von vorher gefärbten, gebleichten und/oder verformten Haaren nicht negativ beeinflussen, und die eine verbesserte Hautverträglichkeit aufweisen. Es ist insbesondere Aufgabe der vorliegenden Anmeldung, das Irritationspotential für die Kopfhaut zu senken und so eine Minimierung des Allergiepotentials gegen Wirkstoffe zu senken, die der Kopfhaut aufgrund von Umwelteinflüssen, UV-Lichteinstrahlung, Hitze oder speziellen Haarbehandlungen zur Regeneration zugeführt werden müssen.

Vollkommen überraschend konnte nun gefunden werden, dass es möglich ist die vorgenannten Aufgaben zu lösen, indem Haarreinigungs- und/oder -konditioniermittel zur Verfügung gestellt werden, die neben einem Tensid und einem speziellen Alkohol mindestens ein funktionalisiertes Tetrapeptid enthalten. Neben der Erhaltung und/oder Steigerung des Volumens und der Fülle insbesondere gefärbter, verformter oder gebleichter Haare konnte die Kopfhautverträglichkeit gesteigert werden.

Ein erster Gegenstand der Erfindung ist daher ein kosmetisches Haarreinigungs- und/oder Konditioniermittel, das
a) mindestens ein oberflächenaktives Mittel, ausgewählt aus der Gruppe der anionischen, amphoteren, zwitterionischen, nichtionischen und/oder kationischen Tenside,
b) mindestens einen geradkettigen oder zyklischen, verzweigten oder unverzweigten aliphatischen C₂-C₆-Alkohol, der an 2 bis 6 Kohlenstoffatomen jeweils eine Hydroxygruppe trägt, und
c) mindestens ein funktionalisiertes Tetrapeptid
enthält.

Es hat sich gezeigt, dass bestimmte funktionalisierte Tetrapeptide in Lage sind, in der Haut, insbesondere der Kopfhaut, die Freisetzung und Ausschüttung des pro-inflammatorischen Neurotransmitters CGRP (Calcitonin Gene Release Peptide) von sensorischen Nervenenden zu reduzieren. Dadurch wird die Toleranzschwelle der Haut, auf irritierende Substanzen zu reagieren, herabgesenkt. Somit werden sowohl Entzündungspotential wie auch Schmerzempfindung der Kopfhaut reduziert. Außerdem kann sich angegriffene, empfindliche Haut beruhigen und so allergienauslösende Substanzen besser tolerieren. Insgesamt führt die Verwendung bestimmter funktionalisierter Tetrapeptide in Mitteln zur Reinigung und/oder Konditionierung von menschlichen Haaren zu einer Verbesserung der Hautverträglichkeit und zu einer Verringerung an Irritationen auf der Kopfhaut.

Die Erfindung betrifft bevorzugt solche Mittel, die - bezogen auf ihr Gesamtgewicht - auf 3 bis 40 Gew.-%, bevorzugt 4 bis 35 Gew.-%, mehr bevorzugt 5 bis 30 Gew.-% und insbesondere 6 bis 25 Gew.-% anionischen, amphoteren und/oder zwitterionischen Tensiden beruhen.

Dabei handelt es sich üblicherweise um Shampoos, die in einer bevorzugten Ausführungsform mindestens ein anionisches Tensid und mindestens ein amphoteres und/oder zwitterionisches Tensid in einem Verhältnis von anionischem(n) Tensid(en) zu amphoterem(n) bzw. zwitterionischem(n) Tensid(en) von 3:1 bis 1:2 bevorzugt von 2:1 bis 1:1, enthalten.

Die Erfindung betrifft ebenfalls bevorzugt solche Mittel, die - bezogen auf ihr Gesamtgewicht - auf 0,1 bis 10 Gew.-%, bevorzugt 0,15 bis 7,5 Gew.-%, mehr bevorzugt 0,2 bis 6,5 Gew.-% und insbesondere 0,25 bis 5 Gew.-% kationischen Tensiden beruhen.

Dabei handelt es sich üblicherweise um leave-on oder rinse-off-Conditioner, Haarkuren, Haarpflege-Sprühemulsionen oder Schaumaerosole.

Als anionische Tenside eignen sich in erfindungsgemäßen Zubereitungen alle für die Verwendung am menschlichen Körper geeignete, insbesondere für die kosmetische Verwendung geeignete, anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- vernetzte und/oder lineare Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate,
- sulfatierte Hydroxyalkylpolyethylen- und/oder Hydroxyalkylenpropylenglykolether gemäß DE-A-37 23 354,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (II), 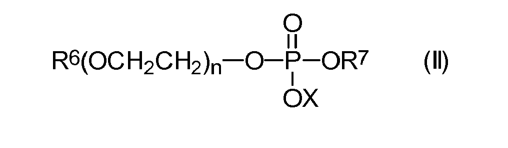 in der R⁶ bevorzugt für einen aliphatischen Kohlenwasserstoffrest mit 8 bis 30 Kohlenstoffatomen, R⁷ für Wasserstoff, einen Rest (CH₂CH₂O)ₙR⁶ oder X, n für Zahlen von 1 bis 10 und X für Wasserstoff, ein Alkali- oder Erdalkalimetall oder NR⁸R⁹R¹⁰R¹¹, mit R⁸ bis R¹¹ unabhängig voneinander stehend für einen C₁ bis C₄ - Kohlenwasserstoffrest, steht,
- sulfatierte Fettsäurealkylenglykolester der Formel (III),

   R¹²CO(AlkO)ₙSO₃M (III)

   in der R¹²CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906.5 beschrieben sind,
- Monoglyceridsulfate und Monoglyceridethersulfate der Formel (IV), 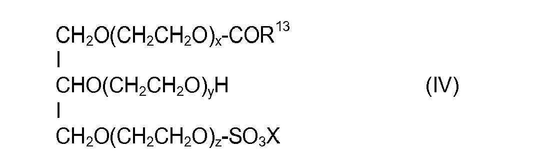 in der R¹³CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vor-zugsweise werden Monoglyceridsulfate der Formel (VIII) eingesetzt, in der R¹³CO für einen linearen Acylrest mit 8 bis 18 Kohlenstoffatomen steht.

Bevorzugte anionische Tenside für alle erfindungsgemäßen Haarreinigungsmittel sind geradkettige und verzweigte Alkylsulfate, Alkylpolyglykolethersulfate und Ethercarbonsäuresalze mit 10 bis 18 C-Atomen in der Alkylgruppe und bis zu 12 Glykolethergruppen im Molekül, Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen.

Besonders bevorzugte anionische Tenside sind die Alkali- oder NH₄-salze vernetzter und/oder linearer Laurylethersulfate und Tridecethsulfate mit einem Ethoxylierungsgrad von 2 bis 4 EO.

Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ -Gruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat.

Ein erfindungsgemäß bevorzugtes zwitterionisches Tensid ist das unter der INCI-Bezeichnung Cocamidopropyl Betaine bekannte Fettsäureamid-Derivat.

Unter amphoteren Tensiden werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe.

Besonders bevorzugte ampholytische Tenside sind das N-Kokosalkylaminopropionat, das Kokosacylaminoethylaminopropionat und das C₁₂ - C₁₈ - Acylsarcosin.

Als Tensidkombination mit der effektivsten Kopfhaut-Schonung hat sich für die erfindungsgemäßen Haarbehandlungsmittel, die als Shampoo konfektioniert werden, eine Kombination aus Alkylethersulfaten und Dialkalimetall Cocoamphodiacetaten in einem Verhältnis von 3:1 bis 1:2, bevorzugt von 2:1 bis 1:1 herausgestellt.

Gemäß einer weiteren bevorzugten Ausführungsform handelt es sich bei dem erfindungsgemäßen Haarbehandlungsmittel um ein Haarkonditioniermittel, das auf kationischen Tensiden basiert.

Diese sind üblicherweise ausgewählt aus der Gruppe der quartären Ammoniumverbindungen, der Esterquats und der Amidoamine.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Behenyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 22 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Der Einsatz von kationischen Tensiden ist nicht auf erfindungsgemäße Haarkonditionierungsmittel beschränkt. Vielmehr können auch erfindungsgemäße Haarreinigungsmittel zur Steigerung der Hautverträglichkeit und Milde ebenfalls kationische Tenside enthalten.

In einer bevorzugten Ausführungsform der Erfindung enthalten die erfindungsgemäßen Haarreinigungs- und Konditioniermittel zur Steigerung der Hautmilde und zur weiteren Verringerung von Hautirritationen weiterhin mindestens ein nichtionisches Tensid und/oder einen nichtionischen Emulgator in einer Menge von 0,1 bis 10 Gew.-%, bevorzugt von 0,15 bis 7,5 Gew.-%, mehr bevorzugt von 0,2 bis 6,5 Gew.-% uns insbesondere von 0,25 bis 5 Gew.-% - bezogen auf das Gesamtgewicht des Mittels.

Erfindungsgemäß geeignete nichtionische Tenside enthalten als hydrophile Gruppe z. B. eine Polyolgruppe, eine Polyalkylenglykolethergruppe oder eine Kombination aus einer Polyol- und einer Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂-C₆- Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl, beispielsweise Rizinusöl-hydriert+40 EO, wie es beispielsweise unter dem Handelsnamen Cremophor CO 455 von der Firma SHC im Handel erhältlich ist,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel (V)

   R¹4CO-(OCH₂CHR¹⁵)_{w}OR¹⁶ (V)

   in der R¹⁴CO für einen linearen oder verzweigten, gesättigten und/oder ungesättigten Acylrest mit 6 bis 22 Kohlenstoffatomen, R¹⁵ für Wasserstoff oder Methyl, R¹⁶ für lineare oder verzweigte Alkylreste mit 1 bis 4 Kohlenstoffatomen und w für Zahlen von 1 bis 20 steht,
- Aminoxide,
- Hydroxymischether,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Fettsäure-N-alkylglucamide,
- Alkylpolygykoside entsprechend der allgemeinen Formel RO-(Z)ₓ wobei R für Alkyl, Z für Zucker sowie x für die Anzahl der Zuckereinheiten steht. Die erfindungsgemäß verwendbaren Alkylpolyglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R im wesentlichen aus C₈- und C₁₀-Alkylgruppen, im wesentlichen aus C₁₂- und C₁₄-Alkylgruppen, im wesentlichen aus C₈- bis C₁₆-Alkylgruppen, im wesentlichen aus C₁₂- bis C₁₆-Alkylgruppen oder im wesentlichen aus C₁₆ bis C₁₈-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 2,0 sind bevorzugt. Ganz besonders bevorzugt sind Alkylglykoside, bei denen x 1,1 bis 1,8 beträgt.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder - alkoholate als Katalysatoren verwendet werden. Die Verwendung von Produkten mit eingeengter Homologenverteilung kann bevorzugt sein.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Als weiteren wesentlichen Inhaltsstoff enthält das erfindungsgemäße Haareinigung- und/oder - konditioniermittel mindestens ein funktionalisiertes Tetrapeptid. Tetrapeptide im Sinne der Erfindung bestehen aus zwei bis acht, bevorzugt aus drei bis sechs, mehr bevorzugt aus vier oder fünf und insbesondere aus vier α-Aminosäuren (alpha-Aminosäuren). Diese sind über Amidbindungen miteinander verknüpft. Somit verbleiben in der Peptid-Hauptkette eine freie Aminogruppe, der sogenannte N-Terminus sowie eine freie Carboxylgruppe, der sogenannte C-Terminus. Gemäß üblicher Darstellungskonvention von Peptiden befinden sich der N-Terminus links und der C-Terminus rechts. Die erfindungsgemäßen Funktionalisierungen bestehen dabei vorzugsweise in Funktionalisierungen am N-Terminus und am C-Terminus.

Bevorzugte Funktionalisierungen am N-Terminus sind dabei Amidinierungen mit geeigneten Carbon- oder Sulfonsäurederivaten, Alkylierungen mit Alkylierungsmitteln sowie in Form von Harnstoff oder Urethanen. Besonders bevorzugt sind Amidierungen mit Carbonsäurenderivaten, wie beispielsweise Acetylchlorid oder Acetylanhydrid. Besonders bevorzugt ist der N-Terminus daher acyliert. Bevorzugte Funktionalisierungen am C-Terminus sind dabei die Veresterung mit Alkoholen sowie Amindinierungen mit Ammoniak, primären oder sekundären Aminen. Besonders bevorzugt ist dabei die Amindierung, bei der der C-Terminus mit Ammoniak zum Carboxamid funktionalisiert ist.

Eine bevorzugte Ausführungsform der vorliegenden Erfindung ist daher dadurch gegeben, dass das erfindungsgemäße Mittel mindestens ein funktionalisiertes Tetrapeptid enthalten, welches dadurch gekennzeichnet ist, dass es als Funktionalisierung am N-Terminus eine Acetylierung und/oder am C-Terminus eine Amidinierung mit Ammoniak aufweist. Besonders bevorzugt ist der N-Terminus acetyliert und der C-Terminus mit Ammoniak amidiniert.

α-Aminosäuren besitzen mit Ausnahme von Glycin zumindest ein asymmetrisches Kohlenstoffzentrum. Um die daraus resultierenden Enantiomere zu unterscheiden, nutzt der Fachmann die sogenannte D-/L-Nomenklatur. Aminosäuren natürlichen Ursprung besitzen üblicherweise die L-Konfiguration. Das funktionalisierte Tetrapeptid besteht bevorzugt aus natürlichen Aminsäuren. Es ist daher erfindungsgemäß bevorzugt, wenn alle Aminosäuren des funktionalisierten Tetrapeptids die L-Konfiguration aufweisen.

Ganz besonders bevorzugt enthält das funktionalisierte Tetrapeptid die Aminosäuren L-Tyrosin, L-Prolin und L-Phenylalanin, wobei es bevorzugt ist, wenn L-Phenylalanin zweimal enthalten ist. Eine ganz besonders bevorzugte Ausführungsform ist dann gegeben, wenn das funktionalisierte Tetrapeptid aus N-acetyliertem L-Tyrosin, L-Prolin, L-Phenylalanin und L-Phenylalaninamid besteht und die Formel (VI) aufweist:

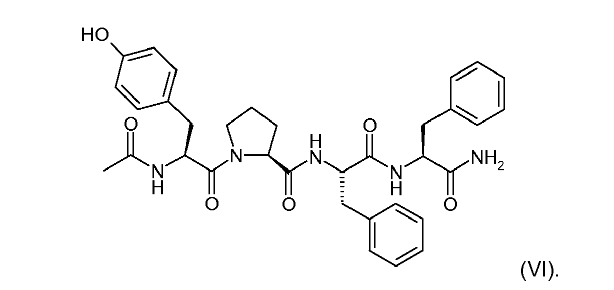

Das Tetrapeptid gemäß Formel (VI) ist auch unter der Kurzschreibweise Ac-YPFF-NH₂ beziehungsweise unter den Bezeichnungen L-Phenylalaninamide-N-Acetyl-L-Tyrosyl-L-Prolyl-L-Phenylalanyl oder N-Acetyl-L-Tyrosyl-L-Prolyl-L-Phenylalanyl-L-Phenylalaninamide bekannt. Die zugehörige INCI-Bezeichnung lautet Acetyl Tetrapeptide-15. Eine besonders bevorzugte Ausführungsform der vorliegenden Erfindung ist daher dann gegeben, wenn das Mittel als funktionalisiertes Tetrapeptid Ac-YPFF-NH₂ enthält.

Erfindungsgemäß bevorzugte Mittel enthalten das funktionalisierte Tetrapeptid in einer Menge von 0,00001 bis 0,5 Gew.-%, bevorzugt von 0,00005 bis 0,25 Gew.-% und insbesondere von 0,0001 bis 0,1 Gew.-%, jeweils bezogen auf das Gesamtgewicht des Mittels.

Ein erfindungsgemäß besonders bevorzugtes Mittel ist **dadurch gekennzeichnet, dass** es Ac-YPFF-NH₂ in einer Menge von 0,00001 bis 0,1 Gew.-%, insbesondere von 0,0001 bis 0,05 Gew.-%, jeweils bezogen auf das Gesamtgewicht des Mittels, enthält.

Das erfindungsgemäße Tetrapeptid kann den Haarbehandlungsmitteln alternativ auch in der Form der Handelsprodukte Skinasensyl^{®} PW LS 9852 oder Skinasensyl^{®} LS 9747 (Laboratoires Serobiologiques) zugefügt werden.

Eine dritte wesentliche Komponente des erfindungsgemäßen Mittels ist ein geradkettiger oder zyklischer, verzweigter oder unverzweigter aliphatischer C₂-C₆-Alkohol, der an 2 bis 6 Kohlenstoffatomen jeweils eine Hydroxygruppe trägt.

Diese Komponenten dienen als Penetrationshilfen für die Haut- und Haarpflegestoffe und steigern das Feuchthaltevermögen von Haut und Haaren. Sie sind bevorzugt ausgewählt aus C₂-C₆-Diolen; Triolen oder aus sogenannten Zuckeralkoholen mit 5 oder 6 Hydroxygruppen.

Beispiele für bevorzugte Komponenten b) sind beispielsweise Glycerin, 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol, Mannitol und/oder Sorbitol.

Insbesondere bevorzugt sind Glycerin, Sorbitol und Mannitol.

Die alkoholische Komponente b) wird in den erfindungsgemäßen Haarreinigungs- und/oder - konditioniermitteln - bezogen auf deren Gesamtgewicht - bevorzugt in Mengen von 0,01 bis 10 Gew.-%, bevorzugt von 0,05 bis 7,5 Gew.-% und insbesondere in Mengen von 0,1 bis 5 Gew.-% enthalten.

Die erfindungsgemäße Aufgabe wird insbesondere durch eine Kombination von Alkylethersulfaten, Alkylamphoacetaten oder -diacetaten, einem funktionalisierten Tetrapeptid und Glycerin, Sorbitol und/oder Mannitol als Wirkstoffkombination gelöst.

Ziel der vorliegenden Erfindung ist es, die Haare und die Kopfhaut mit Haut- und Haarpflegestoffen nachhaltig zu versorgen, ohne dass das Haarvolumen und die Haarfülle einerseits und die sensible Haut andererseits darunter leidet. Die Toleranz der Kopfhaut gegenüber wichtigen Pflegewirkstoffen soll erhöht werden, so dass Haar und Kopfhaut mit diesen versorgt und gepflegt werden können.

Bei den vorgenannten Wirkstoffen handelt es sich um mindestens einen Stoff aus der Gruppe der Proteinhydrolysate, der Vitamine, der Aminosäuren, der Pflanzenextrakte und/oder Ectoin, die in den erfindungsgemäßen Mitteln - bezogen auf deren Gesamtgewicht - in einer Menge von 0,001 bis 10 Gew.-%, bevorzugt in einer Menge von 0,01 bis 7,5 Gew.-% und insbesondere in einer Menge von 0,1 bis 5 Gew.-% eingesetzt werden.

Es kann erfindungsgemäß bevorzugt sein, dass mehrere Stoffe aus der vorgenannten Gruppe der Haut- und Haarpflegewirkstoffe enthalten sind. Erfindungsgemäß bevorzugt für das Erzielen von mehr Haarfülle und Volumen und Kopfhautschonung speziell auf gefärbten, verformten oder gebleichten Haaren ist eine Kombination aus mindestens einem Vitamin und mindestens einem Pflanzenextrakt, einer Aminosäure oder Ectoin oder eine Kombination aus mindestens einem Proteinhydrolysat und mindestens einem Pflanzenextrakt, einer Aminosäure oder Ectoin. Besonders bevorzugt ist die Kombination aus Vitaminen und Aminosäuren, Vitaminen und Ectoin sowie Proteinhydrolysaten und Aminosäuren.

Unter erfindungsgemäß bevorzugten Vitaminen, Provitaminen und Vitaminvorstufen sowie deren Derivaten sind solche Vertreter zu verstehen, die üblicherweise den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäßen Mittel enthalten die Vitamin A-Komponente bevorzugt in Mengen von 0,01-1 Gew.-%, bezogen auf die gesamte Zubereitung.

Zur Vitamin B-Gruppe oder zu dem Vitamin B-Komplex gehören u. a.
Vitamin B₁ (Thiamin)
Vitamin B₂ (Riboflavin)
Vitamin B₃. Unter dieser Bezeichnung werden häufig die Verbindungen Nicotinsäure und Nicotinsäureamid (Niacinamid) geführt.

Erfindungsgemäß bevorzugt ist das Nicotinsäureamid, das in den erfindungsgemäßen Mitteln bevorzugt in Mengen von 0,05 bis 1 Gew.-%, bezogen auf das gesamte Mittel, enthalten ist.

Vitamin B₅ (Pantothensäure und Panthenol). Im Rahmen dieser Gruppe wird bevorzugt das Panthenol eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin B₅-Typs werden bevorzugt in Mengen von 0,05 - 10 Gew.-%, bezogen auf das gesamte Mittel, eingesetzt. Mengen von 0,1 - 5 Gew.-% sind besonders bevorzugt.

Vitamin B₆ (Pyridoxin sowie Pyridoxamin und Pyridoxal).

Die genannten Verbindungen der Vitamin B-Gruppe sind in den erfindungsgemäßen Mitteln bevorzugt in Mengen von 0,01 - 2 Gew.-%, bezogen auf das gesamte Mittel, enthalten. Mengen von 0,03 - 1 Gew.-% sind besonders bevorzugt.

Vitamin C (Ascorbinsäure). Vitamin C wird in den erfindungsgemäß verwendeten Mitteln bevorzugt in Mengen von 0,01 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäß verwendeten Mitteln bevorzugt in Mengen von 0,01-1 Gew.-%, bezogen auf das gesamte Mittel, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3a*S*,4*S*, 6a*R*)-2-Oxohexahydrothienol[3,4-*d*]-imidazol-4-valeriansäure bezeichnet, für die sich aber inzwischen der Trivialname Biotin durchgesetzt hat. Biotin ist in den erfindungsgemäß verwendeten Mitteln bevorzugt in Mengen von 0,0001 bis 1,0 Gew.-%, insbesondere in Mengen von 0,001 bis 0,01 Gew.-% enthalten.

Bevorzugt enthalten die erfindungsgemäßen Mittel Vitamine, Provitamine und Vitaminvorstufen aus den Gruppen A, B, E und H. Selbstverständlich können auch mehrere Vitamine und Vitaminvorstufen gleichzeitig enthalten sein. In einer besonders bevorzugten Ausführungsform der Erfindung enthalten die Haarreinigungs- und/oder -konditioniermittel zur Unterstützung des Feuchthaltevermögens der Kopfhaut und/oder der Haare D-Panthenol, fakultativ in der Mischung mit einem der anderen vorgenannten Vitaminkomponenten.

Erfindungsgemäß geeignete Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch β-Aminosäuren und deren Derivate wie β-Alanin, Anthranilsäure oder Hippursäure eingesetzt werden. Das Molgewicht der erfindungsgemäß einsetzbaren Proteinhydrolysate liegt zwischen 75, dem Molgewicht für Glycin, und 200000, bevorzugt beträgt das Molgewicht 75 bis 50000 und ganz besonders bevorzugt 75 bis 20000 Dalton.

Erfindungsgemäß können Proteinhydrolysate sowohl pflanzlichen als auch tierischen, marinen oder synthetischen Ursprungs in einer Menge von 0,01 bis 10 Gew.-%, bevorzugt von 0,05 bis 5 Gew.-% und insbesondere von 0,1 bis 3 Gew.-% - bezogen auf das Gesamtgewicht des erfindungsgemäßen Mittels - eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex), Kerasol^{®} (Croda) oder ProSina^{®} (Croda) vertrieben.

Erfindungsgemäß geeignete Proteinhydrolysate pflanzlichen Ursprungs sind z. B. Soja-, Mandel-, Erbsen-, Kartoffel-, Reis- und Weizenproteinhydrolysate. Solche Produkte sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Als besonders geeignet hat sich der Zusatz eines Keratin-, Kollagen- oder Seidenproteinhydrolysats zu der erfindungsgemäßen Wirkstoffkombination herausgestellt. Insbesondere bevorzugt in Bezug auf eine Erhöhung des Feuchthaltevermögens ist das Keratinhydrolysat, beispielsweise das unter der Handelsbezeichnung ProSina^{®} von der Firma Croda vertriebene Produkt.

Als weiteren bevorzugten Haut- und/oder Haarpflegewirkstoff können die erfindungsgemäßen Mittel mit besonderem Vorzug eine oder mehrere Aminosäuren enthalten. Aminosäuren wirken ebenfalls heutbefeuchtend und stabilisieren aufgrund ihrer Pufferwirkung den Säuremantel der Haut, d.h. sie dienen dem (Kopf)hautschutz. Erfindungsgemäß geeignete Aminosäuren stammen aus der Gruppe Glycin, Alanin, Valin, Leucin, Isoleucin, Phenylalanin, Tyrosin, Tryptophan, Prolin, Asparaginsäure, Glutaminsäure, Asparagin, Glutamin, Serin, Threonin, Cystein, Methionin, Lysin, Arginin, Histidin, β-Alanin, 4-Aminobuttersäure (GABA), Betain, L-Cystin (L-Cyss), L-Carnitin, L-Citrullin, L-Theanin, 3 ',4 '-Dihydroxy-L-phenylalanin (L-Dopa), 5 '-Hydroxy-L-tryptophan, L-Homocystein, S-Methyl-L-methionin, S-Allyl-L-cystein-sulfoxid (L-Alliin), L-trans-4-Hydroxyprolin, L-5-Oxoprolin (L-Pyroglutaminsäure), L-Phosphoserin, Kreatin, 3-Methyl-L-histidin, L-Ornithin, wobei sowohl die einzelnen Aminosäuren als auch Mischungen eingesetzt werden können.

Bevorzugte erfindungsgemäße Mittel enthalten eine oder mehrere Aminosäuren in engeren Mengenbereichen. Hier sind erfindungsgemäß bevorzugte Haarreinigungsmittel und/oder - konditioniermittel **dadurch gekennzeichnet, dass** sie als Pflegestoff - bezogen auf ihr Gewicht - 0,01 bis 5 Gew.-%, vorzugsweise 0,02 bis 2,5 Gew.-%, besonders bevorzugt 0,05 bis 1,5 Gew.-%, weiter bevorzugt 0,075 bis 1 Gew.-% und insbesondere 0,1 bis 0,25 Gew.-% Aminosäure(n), vorzugsweise aus der Gruppe Glycin und/oder Alanain und/oder Ariginin und/oder Serin und/oder Valin und/oder Lysin und/oder Leucin und/oder Threonin enthalten.

Insbesondere bevorzugt für den Einsatz in den erfindungsgemäßen Mitteln sind die Aminosäuren Glycin, Alanin und/oder Arginin.

Als weiteren bevorzugten Haut- und/oder Haarpflegewirkstoff können die erfindungsgemäßen Mittel mit besonderem Vorzug zur mechanischen und sensorischen Stärkung der Haare Pflanzenextrakte enthalten.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Erfindungsgemäß sind vor allem die Extrakte aus Grünem Tee, Eichenrinde, Echinacea, Brennessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Moringa, Ginseng und Ingwerwurzel bevorzugt.

Erfindungsgemäß ganz besonders bevorzugt aufgrund ihrer haarkräftigenden Wirkung sowie ihrer unterstützenden Wirkung in Bezug auf die Haarfülle sind die Extrakte aus Grünem Tee, Kamille, Mandel, Aloe Vera und Moringa. Insbesondere bevorzugt ist erfindungsgemäß ein Moringa-Extrakt, beispielsweise ein unter dem Handelsnamen Puricare^{®} LS 9658 oder Moringa Oleifera^{®} vertriebenes Produkt.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Mitteln Mischungen aus mehreren, insbesondere aus zwei, verschiedenen Pflanzenextrakten einzusetzen.

Erfindungsgemäß besonders bevorzugte Haarreinigungsmittel und/oder -konditioniermittel sind **dadurch gekennzeichnet, dass** sie - bezogen auf ihr Gewicht - 0,001 bis 10 Gew.-%, bevorzugt 0,005 bis 5 und insbesondere 0,01 bis 2 Gew.-% Pflanzenextrakte (entspricht dem Aktivsubstanzgehalt des Extrakts) enthalten.

Als weiteren Haut- und/oder Haarpflegewirkstoff können die erfindungsgemäßen Mittel bevorzugt Ectoin enthalten. Ectoin ((4S)-2-Methyl-1,4,5,6-Tetrahydropyrimidin-4-Carbonsäure) ist ein zur Gruppe der kompatiblen Solute gehörender Naturstoff. Die stark wasserbindende niedermolekulare organische Verbindung tritt in halophilen Bakterien auf und ermöglicht diesen extremophilen Organismen unter Stressbedingungen zu überleben. Erfindungsgemäß bevorzugte Haarreinigungsmittel und/oder -konditioniermittel sind **dadurch gekennzeichnet, dass** sie - bezogen auf ihr Gewicht - 0,001 bis 10 Gew.-%, vorzugsweise 0,01 bis 5 Gew.-%, besonders bevorzugt 0,05 bis 2,5 Gew.-% und insbesondere 0,1 bis 1 Gew.-% (S)-2-Methyl-1,4,5,6-tetrahydro-4-pyrimidincarbonsäure (Ectoin) sowie die physiologisch verträglichen Salze dieser Verbindung und/oder (S,S)-5-Hydroxy-2-methyl-1,4,5,6-tetrahydro-4-pyrimidincarbonsäure (Hydroxyectoin) sowie die physiologisch verträglichen Salze dieser Verbindung, enthalten.

In einer bevorzugten Ausführungsform der Einfindung enthalten die Haarreinigungs- und/oder - konditioniermittel - bezogen auf ihr Gewicht - neben der erfindungsgemäßen Wirkstoffkombination weiterhin 0,01 bis 2 Gew.-%, bevorzugt 0,05 bis 1,5 Gew.-% und insbesondere 0,1 bis 1 Gew.-% D-Panthenol.

In einer weiteren bevorzugten Ausführungsform der Einfindung enthalten die Haarreinigungsund/oder -konditioniermittel - bezogen auf ihr Gewicht - neben der erfindungsgemäßen Wirkstoffkombination weiterhin 0,01 bis 5 Gew.-%, bevorzugt 0,05 bis 3 Gew.-% und insbesondere 0,1 bis 2 Gew.-% Keratinhydrolysat.

In einer weiteren bevorzugten Ausführungsform der Einfindung enthalten die Haarreinigungsund/oder -konditioniermittel - bezogen auf ihr Gewicht - neben der erfindungsgemäßen Wirkstoffkombination weiterhin eine Mischung aus 0,01 bis 2 Gew.-%, bevorzugt 0,05 bis 1,5 Gew.-% und insbesondere 0,1 bis 1 Gew.-% D-Panthenol und 0,01 bis 2 Gew.-%, bevorzugt 0,05 bis 1,5 Gew.-% und insbesondere 0,1 bis 1 Gew.-% Glycin, Serin und/oder Arginin.

In einer weiteren bevorzugten Ausführungsform der Einfindung enthalten die Haarreinigungs-und/oder -konditioniermittel - bezogen auf ihr Gewicht - neben der erfindungsgemäßen Wirkstoffkombination weiterhin eine Mischung aus 0,01 bis 2 Gew.-%, bevorzugt 0,05 bis 1,5 Gew.-% und insbesondere 0,1 bis 1 Gew.-% D-Panthenol und 0,01 bis 2 Gew.-%, bevorzugt 0,05 bis 1,5 Gew.-% und insbesondere 0,1 bis 1 Gew.-% Ectoin.

In einer weiteren bevorzugten Ausführungsform der Einfindung enthalten die Haarreinigungs-und/oder -konditioniermittel - bezogen auf ihr Gewicht - neben der erfindungsgemäßen Wirkstoffkombination weiterhin eine Mischung aus 0,01 bis 2 Gew.-%, bevorzugt 0,05 bis 1,5 Gew.-% und insbesondere 0,1 bis 1 Gew.-% D-Panthenol und 0,01 bis 2 Gew.-%, bevorzugt 0,05 bis 1,5 Gew.-% und insbesondere 0,1 bis 1 Gew.-% Moringa-Extrakt.

Bei Aufhell- und Blondiermitteln, insbesondere bei solchen, die neutrale Blond-Töne auf dem Haar bewirken, wurde festgestellt, dass sie innerhalb weniger Tage bereits an Leuchtkraft und Helligkeit verlieren. Dies kann durch Proteinabbauprodukte im Haarinneren sowie durch Umwelteinflüsse zustande kommen. Außerdem verlieren blondierte Haare besonders schnell an Volumen und Fülle. Es war somit ein weiterer Aspekt der vorliegenden Erfindung, insbesondere für aufhellende Haarbehandlungsmittel Wirkstoffe bzw. Wirkstoffkombinationen zu finden, die die vorgenannten Nachteile nicht aufweisen.

Durch die Kombination der erfindungsgemäßen Wirkstoffkombination mit
- einem kosmetisch akzeptablen Peroxid und/oder Percarbamid und
- Zitronensäure, Ascorbinsäure, iso-Ascorbinsäure und/oder Phosphorsäure
konnte auch dieses Ziel erfüllt werden, denn die kombinierten aufhellenden oder blondierenden Haarreinigungs- und/oder -konditioniermittel verleihen den damit behandelten Haaren mehr Fülle und Volumen. Gleichzeitig erhöhen sie die Hautverträglichkeit, indem das Irritationspotential der Peroxidkomponente durch das Tetrapeptid kompensiert wird. Darüber hinaus konnte festgestellt werden, dass durch diese erfindungsgemäß bevorzugten aufhellenden oder blondierenden Haarreinigungs- und/oder -konditioniermittel unerwünschte Farbschleier, insbesondere auf blondierten Haaren mit neutralen Blond-Tönen, schon nach einigen wenigen Anwendungen des Mittels entfernt und die Neutralität des Haarfarbtons wiederhergestellt werden kann.

In einer besonders bevorzugten weiteren Ausführungsform der Erfindung enthalten die erfindungsgemäßen Haarreinigungs- und/oder Konditioniermittel daher weiterhin
- ein kosmetisch akzeptables Peroxid und/oder Percarbamid und
- Zitronensäure, Ascorbinsäure, iso-Ascorbinsäure und/oder Phosphorsäure.

Alle vorgenannten Tenside und Haar- und/oder Hautpflegestoffe eignen sich gleichermaßen auch für den Einsatz in den erfindungsgemäßen aufhellenden oder blondierenden Haarreinigungs-und/oder -konditioniermitteln.

Aufhellmittel sind üblicherweise chemische Oxidationsmittel, mit deren Hilfe natürliche und künstliche Farbstoffe im Haar zerstört und das Haar damit gebleicht wird. Als Aufhellmittel kommen daher Persulfate, Peroxomonosulfate, Chlorite, Hypochlorite und insbesondere H₂O₂ oder dessen Anlagerungsprodukte an Harnstoff, Melamin sowie Natriumborat in Frage.

Bevorzugt wird als Oxidationsmittel Wasserstoffperoxid und/oder Percarbamid eingesetzt. Bevorzugt beträgt die Menge an Wasserstoffperoxid und/oder Percarbamid im erfindungsgemäßen Haarreinigungs- und/oder -konditioniermittel 0,01 bis 5 Gew.-%, bevorzugt 0,05 bis 3 Gew.-% und insbesondere 0,1 bis 2 Gew.-%, jeweils bezogen auf das Gesamtgewicht des Mittels.

Die erfindungsgemäßen aufhellenden Haarreinigungs- und -konditioniermittel enthalten weiterhin zur Unterstützung der vorgenannten Wirkungen eine Säurekomponente, ausgewählt aus Zitronensäure, Ascorbinsäure, iso-Ascorbinsäure und/oder Phosphorsäure. Erfindungsgemäß bevorzugt ist der Einsatz von Zitronensäure.

Die Säurekomponente wird den erfindungsgemäßen Mitteln - bezogen auf deren Gesamtgewicht
- in einer Menge von 0,01 bis 5 Gew.-%, Bevorzugt von 0,05 bis 3 Gew.-% und ins besondere von 0,1 bis 2 Gew.-%, zugegeben.

Alle erfindungsgemäßen Mittel weisen einen pH-Wert im Bereich von 2 bis 9 auf. Erfindungsgemäß bevorzugt für die Steigerung der Hautverträglichkeit ist ein pH-Wert im neutralen bis aciden Bereich von 2,5 bis 7. Insbesondere bevorzugt und besonders hautverträglich sind die Mittel in einem pH-Bereich von 3 bis 6.

Neben den zwingenden Bestandteilen der erfindungsgemäßen Wirkstoffkombination können die erfindungsgemäßen Haarreinigungs - und/oder -konditioniermittel in einer bevorzugten Ausführungsform noch weitere Haarpflegestoffe enthalten, die die erfindungsgemäße Wirkung noch weiter steigern bzw. unterstützen können.

Diese bevorzugten weiteren Wirkstoffe sind ausgewählt aus der Gruppe der natürlichen und synthetischen Ölkomponenten, der Antischuppenwirkstoffe, der kationischen Polymere, der UV-Filter und/oder der Silikone.

Erfindungsgemäß einsetzbar sind vorzugsweise kationische Polymere. Unter erfindungsgemäß geeigneten kationischen Polymeren sind Polymere zu verstehen, welche in der Haupt- und/oder Seitenkette Gruppen aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (VI), 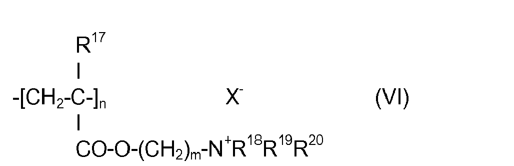 in der R¹⁷ = -H oder -CH₃ ist, R¹⁸, R¹⁹ und R²⁰ unabhängig voneinander ausgewählt sind aus C₁₋₄-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (III) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
- R¹⁷ steht für eine Methylgruppe
- R¹⁸, R¹⁹ und R²⁰ stehen für Methylgruppen
- m hat den Wert 2.

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein geeignetes Homopolymer ist das, gewünschtenfalls vernetzte, Poly(methacryloyloxyethyltrimethylammoniumchlorid) mit der INCI-Bezeichnung Polyquaternium-37. Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten:
Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und
Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (VI) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Weitere bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- hydrophob modifizierte Cellulosederivate, beispielsweise die unter dem Handelsnamen SoftCat^{®} vertriebenen kationischen Polymere,
- kationische Alkylpolyglycoside,
- kationiserter Honig, beispielsweise das Handelsprodukt Honeyquat^{®} 50,
- kationische Guar-Derivate, wie insbesondere die unter den Handelsnamen Cos-media^{®}Guar und Jaguar^{®} vertriebenen Produkte,
- Polysiloxane mit quaternären Gruppen, wie beispielsweise die im Handel erhältlichen erhältlichen Produkte Q2-7224 (Hersteller: Dow Corning; ein stabilisiertes Trimethylsilylamodimethicon), Dow Corning^{®} 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Goldschmidt; diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind. Chitosane sind deacetylierte Chitine, die in unterschiedlichen Deacetylierungsgraden und unterschiedlichen Abbaugraden (Molekulargewichten) im Handel erhältlich sind.

In einer besonders bevorzugten Ausführungsform der Erfindung enthalten die Mittel weiterhin ein kationisches Polymer zur Steigerung der Abscheidung kosmetischer Haut- und Haarpflegewirkstoffe sowie zur Haut- und Haarkonditionierung. Bei diesem handelt es sich bevorzugt um ein kationisches Guar-Derivat und/oder Polyquaternium-7 (Merquat 550), Polyquaternium-6, Polyquaternium-10, Polyquaternium-67 (SoftCat^{®}-Polymere) und oder Salcare^{®} SC 95 bzw. Salcare^{®} SC 96. Das oder die kationische(n) Polymer(e) wird (werden) in den erfindungsgemäßen Mitteln in einer Menge von 0,01 bis 10 Gew.-%, bevorzugt von 0,05 bis 5 Gew.-% und insbesondere von 0,1 bis 3 Gew.-% - bezogen auf das Gesamtgewicht des Mittels - eingesetzt.

Eine weitere erfindungsgemäß bevorzugte Komponente ist ein Öl, das unter natürlichen und synthetischen Ölkomponenten und/oder Fettstoffen ausgewählt sein kann.

Insbesondere die erfindungsgemäßen Konditioniermittel enthalten mindestens eine Öl- und/oder Fettkomponente.

Als natürliche (pflanzliche) Öle werden üblicherweise Triglyceride und Mischungen von Triglyceriden eingesetzt. Bevorzugte natürliche Öle im Sinne der Erfindung sind Kokosnussöl, (süßes) Mandelöl, Walnussöl, Pfirsichkernöl, Aprikosenkernöl, Avocadoöl, Teebaumöl (Tea Tree Oil), Sojaöl, Sesamöl, Sonnenblumenöl, Tsubakiöl, Nachtkerzenöl, Reiskleieöl, Palmkernöl, Mangokernöl, Wiesenschaumkrautöl, Distelöl, Macadamianussöl, Traubenkernöl, Amaranthsamenöl, Arganöl, Bambusöl, Olivenöl, Weizenkeimöl, Kürbiskernöl, Malvenöl, Haselnussöl, Safloröl, Canolaöl, Sasanquaöl, Jojobaöl, Kakaoabutter und Shea-Butter.

Als mineralische Öle kommen insbesondere Mineralöle, Paraffin- und Isoparaffinöle sowie synthetische Kohlenwasserstoffe zum Einsatz. Ein erfindungsgemäß einsetzbarer Kohlenwasserstoff ist beispielsweise das als Handelsprodukt erhältliche 1,3-Di-(2-ethylhexyl)-cyclohexan (Cetiol^{®} S).

Als synthetische Öle kommen Silikonverbindungen in Betracht. Die Silikone werden an späterer Stelle dieser Anmeldung ausführlich beschrieben.

Als Ölkomponente kann weiterhin ein Dialkylether dienen.

Erfindungsgemäß einsetzbare Dialkylether sind insbesondere Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyln-undecylether sowie Di-tert.-butylether, Di-iso-pentylether, Di-3-ethyldecylether, tert.-Butyl-n-octylether, iso-Pentyl-n-octylether und 2-Methylpentyl-n-octylether.

Erfindungsgemäß besonders bevorzugt ist der Di-n-octylether, der im Handel unter der Bezeichnung Cetiol^{®} OE erhältlich ist.

Die erfindungsgemäßen Mittel enthalten die Ölkomponente(n) bevorzugt in einem Mengenbereich von 0,1 bis 7 Gew.-%, insbesondere von 0,25 bis 5 Gew.-%, bezogen auf das Gesamtgewicht des Mittels.

In einer weiteren bevorzugten Ausführungsform der Erfindung kann die Wirkung der erfindungsgemäßen Wirkstoffkombination durch weitere Fettstoffe noch weiter optimiert werden. Unter weiteren Fettstoffen sind zu verstehen Fettsäuren, Fettalkohole sowie natürliche und synthetische Wachse, welche sowohl in fester Form als auch flüssig in wässriger Dispersion vorliegen können.

Als Fettsäuren können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol^{®} 871 und Emersol^{®} 875, und Isopalmitinsäuren wie das Handelsprodukt Edenor^{®} IP 95, sowie alle weiteren unter den Handelsbezeichnungen Edenor^{®} (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Die Einsatzmenge beträgt dabei 0,1 - 15 Gew.%, bezogen auf das gesamte Mittel. In einer bevorzugten Ausführungsform beträgt die Menge 0,5 - 10 Gew.%, wobei ganz besonders vorteilhaft Mengen von 1 - 5 Gew.% sind.

Als Fettalkohole können eingesetzt werden gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂- Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol^{®}, z.B. Stenol^{®} 1618 oder Lanette^{®}, z.B. Lanette^{®} O oder Lorol^{®}, z.B. Lorol^{®} C8, Lorol^{®} C14, Lorol^{®} C18, Lorol^{®} C8-18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, Isofol^{®} 24, Isofol^{®} 36, Isocarb^{®} 12, Isocarb^{®} 16 oder Isocarb^{®} 24 käuflich zu erwerben. Selbstverständlich können erfindungsgemäß auch Wollwachsalkohole, wie sie beispielsweise unter den Bezeichnungen Corona^{®}, White Swan^{®}, Coronet^{®} oder Fluilan^{®} käuflich zu erwerben sind, eingesetzt werden.

Die Fettalkohole werden in Mengen von 0,1 - 20 Gew.-%, bezogen auf die gesamte Zubereitung, bevorzugt in Mengen von 0,1 - 10 Gew.-% eingesetzt.

Als natürliche oder synthetische Wachse können erfindungsgemäß eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Weitere Fettstoffe sind beispielsweise
- Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkohol-caprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Din-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, Glycerincarbonat oder Dicaprylylcarbonat (Cetiol^{®} CC),
- ethoxylierte oder nicht ethoxylierte Mono,- Di- und Trifettsäureester von gesättigten und/oder ungesättigten linearen und/oder verzweigten Fettsäuren mit Glycerin, wie beispielsweise Monomuls^{®} 90-018, Monomuls^{®} 90-L12, Cetiol^{®} HE oder Cutina^{®} MD.

Die Einsatzmenge der weiteren Fettstoffe beträgt 0,1 -20 Gew. % bezogen auf das gesamte Mittel. Bevorzugt sind 0,1 - 10 Gew.% und besonders bevorzugt 0,1 - 5 Gew.%, bezogen auf das gesamte Mittel.

Die Gesamtmenge an Öl- und Fettkomponenten in den erfindungsgemäßen Mitteln beträgt üblicherweise 0,01 - 15 Gew.-%, bezogen auf das gesamte Mittel. Mengen von 0,05 bis 10 Gew.-% und insbesondere von 0,05- 5 Gew.-% sind erfindungsgemäß bevorzugt.

Antischuppenmittel tragen ebenfalls zur Verbesserung der erfindungsgemäßen Mittel bei, denn mit der Verringerung oder Inhibierung von Kopfschuppen geht auch eine bessere Hautverträglichkeit und ein geringeres Irritationspotential der Kopfhaut einher. Erfindungsgemäß bevorzugte Mittel enthalten somit weiterhin mindestens einen kosmetisch akzeptablen Antischuppenwirkstoff. Dieser wird den erfindungsgemäßen Zusammensetzungen in einer Menge von 0,05 bis 5 Gew.-%, bevorzugt von 0,1 bis 3,0 Gew.-% und insbesondere von 0,3 bis 2,0 Gew.-% zugegeben (bezogen auf das gesamte Mittel) und ist ausgewählt aus Piroctone Olamine, Climbazol, Zink Pyrithion, Ketoconazole, Salicylsäure, Schwefel, Selensulfide, Teerpräparaten, Undecensäurederivaten, Klettenwurzelextrakten, Pappelextrakten, Brennesselextrakten, Walnussschalenextrakten, Birkenextrakten, Weidenrindenextrakten, Rosmarinextrakten und/oder Arnikaextrakten.

Erfindungsgemäß bevorzugt sind Salicylsäure, Climbazol, Zink Pyrithion und Piroctone Olamine. Weiterhin kann in einer bevorzugten Ausführungsform der Erfindung ein erfindungsgemäßes Mittel auch UV - Filter (I) enthalten, da UV-Filter die Kopfhaut vor erhöhter Sonnenstrahleneinwirkung schützen. Des weiteren ist es bekannt, dass bestimmte Wirkstoffe in der Kombination mit erhöhter Sonnenlicht bzw. UV-Lichteinstrahlung Hautirritationen hervorrufen können, daher ist es erfindungsgemäß bevorzugt, wenn die Mittel weiterhin einen UV-Filter enthalten.

Die erfindungsgemäß zu verwendenden UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280nm)-Bereich liegt. UV-Filter mit einem Absorptionsmaximum im UVB-Bereich, insbesondere im Bereich von etwa 280 bis etwa 300 nm, sind besonders bevorzugt.

Die erfindungsgemäß verwendeten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Bevorzugt sind solche UV-Filter, deren molarer Extinktionskoeffizient am Absorptionsmaximum oberhalb von 15 000, insbesondere oberhalb von 20000, liegt.

Weiterhin wurde gefunden, dass bei strukturell ähnlichen UV-Filtern in vielen Fällen die wasserunlösliche Verbindung im Rahmen der erfindungsgemäßen Lehre die höhere Wirkung gegenüber solchen wasserlöslichen Verbindungen aufweist, die sich von ihr durch eine oder mehrere zusätzlich ionische Gruppen unterscheiden. Als wasserunlöslich sind im Rahmen der Erfindung solche UV-Filter zu verstehen, die sich bei 20 °C zu nicht mehr als 1 Gew.-%, insbesondere zu nicht mehr als 0,1 Gew.-%, in Wasser lösen. Weiterhin sollten diese Verbindungen in üblichen kosmetischen Ölkomponenten bei Raumtemperatur zu mindestens 0,1, insbesondere zu mindestens 1 Gew.-% löslich sein). Die Verwendung wasserunlöslicher UV-Filter kann daher erfindungsgemäß bevorzugt sein.

Gemäß einer weiteren Ausführungsform der Erfindung sind solche UV-Filter bevorzugt, die eine kationische Gruppe, insbesondere eine quartäre Ammoniumgruppe, aufweisen.

Zwei bevorzugte UV-Filter mit kationischen Gruppen sind die als Handelsprodukte erhältlichen Verbindungen Zimtsäureamidopropyl-trimethylammoniumchlorid (Incroquat^{®}UV-283) und Dodecyl-dimethylaminobenzamidopropyl-dimethylammoniumtosylat (Escalol^{®} HP 610).

Selbstverständlich umfasst die erfindungsgemäße Lehre auch die Verwendung einer Kombination von mehreren UV-Filtern. Im Rahmen dieser Ausführungsform ist die Kombination mindestens eines wasserunlöslichen UV-Filters mit mindestens einem UV-Filter mit einer kationischen Gruppe bevorzugt.

Die UV-Filter (I) sind in den erfindungsgemäßen Mitteln üblicherweise in Mengen 0,1-5 Gew.-%, bezogen auf das gesamte Mittel, enthalten. Mengen von 0,4-2,5 Gew.-% sind bevorzugt.

Insbesondere bevorzugt sind erfindungsgemäße Haarbehandlungsmittel, die mindestens ein Silicon enthalten. Silikone bewirken auf dem Haar ausgezeichnete konditionierende Eigenschaften. Insbesondere bewirken sie eine bessere Kämmbarkeit der Haar in nassem und trockenem Zustand und wirken sich in vielen Fällen positiv auf den Haargriff und die Weichheit der Haare aus.

Es ist daher erfindungsgemäß erstrebenswert, insbesondere den Haarkonditioniermitteln mindestens eine Silikonkomponente hinzuzufügen. Diese(s) (ist) sind ausgewählt unter:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)-Polyoxyalkylen(B)-Blockcopoylmeren des Typs (A-B)ₙ mit n > 3;
(iv) gepfropften Siliconpolymeren mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(v) gepfropften Siliconpolymeren mit Polysiloxan- Grundgerüst, auf das nicht siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon enthält;
(vi) oder deren Gemischen.

Die vorgenannten Silikonkomponenten werden den erfindungsgemäßen Mitteln - bezogen auf deren Gesamtgewicht - in einer Menge von 0,1 bis 10 Gew.-%, bevorzugt von 0,2 bis 7,5 Gew.-% und insbesondere von 0,3 bis 5 Gew.-% (bezogen auf den Aktivsubstanzgehalt des Silikons) hinzugefügt.

In einer weiteren bevorzugten Ausführungsform können die erfindungsgemäßen Mittel Emulgatoren (F) enthalten. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Erfindungsgemäß verwendbare Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®} PGPH),
- Lineare und verzweigte Fettsäuren mit 8 bis 30 C - Atomen und deren Na-, K-, Ammonium-, Ca-, Mg- und Zn - Salze.

Die erfindungsgemäßen Mittel enthalten die Emulgatoren bevorzugt in Mengen von 0,1 - 25 Gew.-%, insbesondere 0,5 - 15 Gew.-%, bezogen auf das gesamte Mittel.

Bevorzugt können die erfindungsgemäßen Zusammensetzungen mindestens einen nichtionogenen Emulgator mit einem HLB-Wert von 8 bis 18 enthalten. Nichtionogene Emulgatoren mit einem HLB-Wert von 10 - 15 können erfindungsgemäß besonders bevorzugt sein.

Ein zweiter Gegenstand der vorliegenden Erfindung ist die Verwendung der erfindungsgemäßen Haarreinigungs- und/oder -konditioniermittel zur Steigerung des Haarvolumens sowie zur Verbesserung der Kopfhautverträglichkeit.

Ein dritter Gegenstand der vorliegenden Erfindung ist die Verwendung der erfindungsgemäßen Haarreinigungs- und/oder -konditioniermittel zur Erhaltung und/oder Verstärkung eines neutralen Blondtons durch kontinuierliche Verwendung des Mittels.

Bezüglich bevorzugter Ausführungsformen der erfindungsgemäßen Verwendungen und des erfindungsgemäßen Verfahrens gilt mutatis mutandis das zu den erfindungsgemäßen Mitteln Gesagte.

Die folgenden Beispiele erläutern die Erfindung, ohne sie jedoch darauf einzuschränken:

Alle Angaben sind - sofern nicht anders angegeben - in Gewichtsprozent.

### Beispiele:

### Haarpflegeshampoos 1 bis 4 und aufhellendes Haarpflegeshampoo 5:

| | **1** | **2** | **3** | **4** | **5** |
|---|---|---|---|---|---|
| Ethoxyliertes Fettalkoholsulfat (C12-C14, 2 EO; 70% AS) | 15 | 15 | 15 | 15 | 15 |
| Disodium Cocoamphodiacetate | 7 | - | 3 | - | 7 |
| Cocamidopropylbetain | - | 5 | 2 | 5 | - |
| Plantacare^{®4} 818 UP | - | 4 | 4 | 4 | - |
| Euperlan^{®}PK 3000 AM¹ | 2,5 | - | - | - | 2,5 |
| Coconut Glycerides + 7,3 EO | 0,5 | 0,5 | 0,5 | 0,5 | 0,5 |
| Cutina HR^{®2} | 0,1 | - | - | - | 0,1 |
| D-Panthenol | 0,5 | 0,5 | - | 0,5 | 0,5 |
| Skinasensyl^{®} PW LS 9852³ | 0,5 | 0,3 | 0,75 | 0,5 | 0,5 |
| ProSina^{®5} | - | - | 0,5 | - | - |
| Glycin | - | 0,2 | - | - | - |
| Ectoin | - | - | - | 0,2 | - |
| Zitronensäure | 0,5 | 0,5 | 0,5 | 0,5 | 0,5 |
| Wasserstoffperoxid | - | - | - | - | 0,2 |
| Trinatriumcitrat Dihydrat | - | - | - | - | 0,2 |
| Salicylsäure | 0,2 | - | - | - | 0,2 |
| Glycerin | 0,15 | 0,1 | - | - | 0,2 |
| Mannitol | - | - | 0,1 | - | |
| Sorbitol | - | - | - | 0,1 | |
| Sodium Benzoate | 0,4 | 0,4 | 0,4 | 0,4 | 0,4 |
| NaCl | 1,0 | 1,0 | 1,0 | 1,0 | 1,0 |
| Parfum | q.s. | q.s. | q.s. | q.s. | q.s. |
| Wasser | ad 100 | ad 100 | ad 100 | ad 100 | ad 100 |

### Rinse-off Conditioner I:

| | |
|---|---|
| Cetearyl Alcohol | 2,5 |
| Eumulgin^{®} B2⁶ | 0,5 |
| Genamin KDMP^{®7} | 3,0 |
| Cutina CP^{®8} | 0,5 |
| Natrosol 250 HR^{®9} | 0,5 |
| Timiron Super Silver^{®10} | 0,1 |
| Mannitol | 0,1 |
| Glycin | 0,2 |
| Methylparaben | 0,3 |
| Phenoxyethanol | 0,4 |
| D-Panthenol | 0,5 |
| Skinasensyl^{®3} PW LS 9852 | 1,0 |
| Parfum | q.s |
| Wasser | ad 100 |

Ein Rinse-off-Conditioner II mit aufhellender Wirkung enthält zusätzlich 0,4 Gew.-% Wasserstoffperoxid und 0,2 Gew.-% Citronensäure.

### Leave-on Conditioner III; Sprüh-Emulsion:

| | |
|---|---|
| Ethanol 96% DEP vergällt | 10 |
| Dow Corning 5330 Fluid^{®11} | 2,0 |
| Dow Corning 949^{®12} | 5,0 |
| PEG-40 Hydrogenated Castor Oil | 1,0 |
| Sodium Benzoate | 0,1 |
| Sorbitol | 0,1 |
| ProSina^{®5} | 0,2 |
| Skinasensyl^{®3} PW LS 9852 | 1,0 |
| Parfum | q.s |
| Wasser | ad 100 |

Ein Leave-on-Conditioner IV mit aufhellender Wirkung enthält zusätzlich 0,1 Gew.-% Wasserstoffperoxid und 0,4 Gew.-% Citronensäure.

### Leave-on-Conditioner V; Schaumaerosol:

| | |
|---|---|
| Salcare^{®13} SC 96 | 2,0 |
| Genamin^{®14} CTAC | 1,5 |
| Dow Corning^{®15} 939 | 0,5 |
| D-Panthenol | 0,2 |
| Glycin | 0,1 |
| Skinasensyl^{®3} PW LS 9852 | 0,5 |
| Methylparaben | 0,2 |
| Phenoxyethanol | 0,4 |
| Glycerin | 0,15 |
| Isobutan | 6,0 |
| Parfum | q.s |
| Wasser | ad 100 |

Ein Leave-on-Conditioner VI mit aufhellender Wirkung enthält zusätzlich 0,2 Gew.-% Wasserstoffperoxid und 0,3 Gew.-% Citronensäure.

Es wurden die folgenden Handelsprodukte eingesetzt:
- 1: INCI-Bezeichnung: Aqua, Glycol Distearate, Glycerin, Laureth-4, Cocamidopropyl Betaine, Formic Acid (Solids: 41-45%); Cognis
- 2: INCI-Bezeichnung: Hydrogenated Castor Oil; Cognis
- 3: INCI-Bezeichnung: Acyetyl Tetrapeptide-15 and Sodium Citrate (AS 0,1 bis 1%); Laboratoires Serobiologiques
- 4: INCI-Bezeichnung: Coco-Glucoside (AS 51-53%); Cognis
- 5: INCI-Bezeichnung: Aqua, Hydrolyzed Keratin; Croda
- 6: INCI-Bezeichnung: Ceteareth-20; Cognis
- 7: INCI-Bezeichnung: Behentrimonium Chloride; Cognis
- 8: INCI-Bezeichnung: Cetyl Palmitate; Cognis
- 9: INCI-Bezeichnung: Hydroxyethylcellulose; Hercules
- 10: INCI-Bezeichnung: Mica; CI 77891; Merck
- 11: INCI-Bezeichnung: PEG/PPG-15/15-Dimethicone; Dow Corning
- 12: INCI-Bezeichnung: Amodimethicone, Cetrimonium Chloride, Trideceth-12; Dow Corning
- 13: INCI-Bezeichnung: Polyquaternium-37, Propylene Glycol, Dicaprylate/Dicaprate, PPG-1 Trideceth-6; Ciba
- 14: INCI-Bezeichnung: Cetrimonium Chloride; Cognis
- 15: INCI-Bezeichnung: Amodimethicone, Cetrimonium Chloride, Trideceth-12; Dow Corning

## Patentansprüche

1. Kosmetisches Haarreinigungs- und/oder -konditioniermittel, enthaltend
a) mindestens ein oberflächenaktives Mittel, ausgewählt aus der Gruppe der anionischen, amphoteren, zwitterionischen, nichtionischen und/oder kationischen Tenside,
b) mindestens einen geradkettigen oder zyklischen, verzweigten oder unverzweigten aliphatischen C₂-C₆-Alkohol, der an 2 bis 6 Kohlenstoffatomen jeweils eine Hydroxygruppe trägt, und
c) mindestens ein funktionalisiertes Tetrapeptid.

2. Mittel nach Anspruch 1, **dadurch gekennzeichnet, dass** es - bezogen auf sein Gewicht - 3 bis 40 Gew.-%, bevorzugt 4 bis 35 Gew.-%, mehr bevorzugt 5 bis 30 Gew.-% und insbesondere 6 bis 25 Gew.-% anionische, amphotere und/oder zwitterionische Tenside umfasst.

3. Mittel nach Anspruch 1, **dadurch gekennzeichnet, dass** es - bezogen auf sein Gewicht - 0,1 bis 10 Gew.-%, bevorzugt 0,15 bis 7,5 Gew.-%, mehr bevorzugt 0,2 bis 6,5 Gew.-% und insbesondere 0,25 bis 5 Gew.-% mindestens eines kationischen Tensids umfasst.

4. Mittel nach einem der Ansprüche 1 bis 3, **dadurch gekennzeichnet, dass** es 0,1 bis 10 Gew.-%, bevorzugt 0,15 bis 7,5 Gew.-%, mehr bevorzugt 0,2 bis 6,5 Gew.-% und insbesondere 0,25 bis 5 Gew.-% mindestens eines nichtionischen Tensids und/oder eines nichtionischen Emulgators umfasst.

5. Mittel nach einem der Ansprüche 1 bis 4, **dadurch gekennzeichnet, dass** das funktionalisierte Tetrapeptid c) als Funktionalisierungen am N-Terminus eine Acetylierung und/oder am C-Terminus eine Amidierung mit Ammoniak aufweist.

6. Mittel nach einem der Ansprüche 1 bis 5, **dadurch gekennzeichnet, dass** alle Aminosäuren des funktionalisierten Tetrapeptids c) eine L-Konfiguration aufweisen.

7. Mittel nach einem der Ansprüche 1 bis 6, **dadurch gekennzeichnet, dass** es als funktionalisiertes Tetrapeptid c) L-Phenylalaninamide-N-Acetyl-L-Tyrosyl-L-Prolyl-L-Phenylalanyl (Acetyl Tetrapeptide-15) enthält.

8. Mittel nach einem der Ansprüche 1 bis 7, **dadurch gekennzeichnet, dass** es die Komponente c) - bezogen auf sein Gewicht - in einer Menge von 0,00001 bis 0,5 Gew.-%, bevorzugt von 0,00005 bis 0,25 Gew.-% und insbesondere in einer Menge von 0,0001 bis 0,1 Gew.-% enthält.

9. Mittel nach einem der Ansprüche 1 bis 8, **dadurch gekennzeichnet, dass** es weiterhin mindestens einen Haarpflegestoff aus der Gruppe der Proteinhydrolysate und/oder der Vitamine und/oder der Aminosäuren und/oder der Pflanzenextrakte und/oder Ectoin enthält.

10. Mittel nach Anspruch 9, **dadurch gekennzeichnet, dass** es mindestens einen Haarpflegestoff auf der Gruppe Panthenol, Keratinhydrolysat, Glycin, Serin, Arginin, Moringa Oleifera und/oder Ectoin enthält.

11. Mittel nach einem der Ansprüche 1 bis 10, **dadurch gekennzeichnet, dass** es als Shampoo, Leave-in oder Rinse-off Conditioner, Haarkur, Haarpflege-Sprühemulsion oder Schaumaerosol konfektioniert ist.

12. Mittel nach einem der Ansprüche 1 bis 11, **dadurch gekennzeichnet, dass** es zusätzlich eine Kombination aus
a) einem kosmetisch akzeptablen Peroxid und/oder Percarbamid und
b) Zitronensäure, Ascorbinsäure, Iso-Ascorbinsäure und/oder Phosphorsäure enthält.

13. Mittel nach Anspruch 12, **dadurch gekennzeichnet, dass** es einen pH-Wert im Bereich von 2 bis 9, bevorzugt von 2,5 bis 7 und insbesondere von 3 bis 6 aufweist.

14. Verwendung eines Mittels nach einem der Ansprüche 1 bis 13 zur Steigerung des Haarvolumens und der Haarfülle sowie zur Verbesserung der Kopfhautverträglichkeit.

15. Verwendung eines Mittels nach einem der Ansprüche 12 oder 13 Anspruch 12 zur Erhaltung bzw. Verstärkung eines neutralen Blondtons.
